# EUROPEAN PATENT APPLICATION

(11) **EP 4 019 067 A1**
(43) Date of publication of application: **29.06.2022**
(21) Application number: 20216615.3
(22) Date of filing: 22.12.2020
(51) Int. Cl.: A61M 5/32

(54) **CANNULA FOR ADMINISTERING A LOCAL ANESTHETIC FOR CASTRATION OF A PIGLET**

(71) Applicant: Technische Universität München, 80333 München (DE); Ludwig-Maximilians-Universität München, 80539 München (DE)
(72) Inventor: BAUMGARTNER, Christine, 80336 Munich (DE); SALLER, Anna, 82205 Gilching (DE); WERNER, Julia, 81543 Munich (DE); REISER, Judith, 80686 Munich (DE); FISCHER, Johannes, 85354 Freising (DE); ZÖLS, Susanne, 85778 Haimhausen (DE); SENF, Steffanie, 80469 Munich (DE); DEFFNER, Pauline, 85221 Dachau (DE); ABENDSCHÖN, Nora, 80687 Munich (DE)
(74) Representative: Lucke, Andreas

(57) **Abstract**

Disclosed herein is a cannula for administering a local anesthetic for castration of a piglet. The cannula comprises a hollow shaft extending from a proximal end to a distal end, wherein the distal end of the hollow shaft comprises a needle tip for inserting the distal end through a scrotal skin of a scrotum of the piglet into a testicle of the piglet. A depth reference is arranged along the hollow shaft, the depth reference indicating a depth up to which the hollow shaft is to be inserted into the scrotum. The hollow shaft comprises a plurality of openings for releasing the local anesthetic from the hollow shaft through each of the openings simultaneously. The plurality of openings comprises a distal opening arranged at the distal end of the hollow shaft, a distal lateral opening arranged on a side face of the hollow shaft, and a proximal lateral opening arranged on a side face of the hollow shaft. A center of the distal lateral opening is arranged at a first distance from the needle tip, the first distance being between 2.5 mm and 6.0 mm. The proximal lateral opening is arranged between the depth reference and the distal lateral opening and a center of the proximal lateral opening is arranged at a second distance from the depth reference, the second distance being between 0.5 mm and 2.0 mm.

## Description

### FIELD OF THE INVENTION

The present invention is in the fields of veterinary medicine and agriculture. In particular, the invention relates to a cannula for administering a local anesthetic for castration of a piglet.

### BACKGROUND

Castration of male piglets is for example performed in pig farms in order to avoid issues associated with adult boars such as a characteristic smell of boar meat (boar taint) and aggressive behavior of the animals. As a result of intense cost pressure in this industry, castration has so far often been performed without anesthetizing the piglets, which - contrary to the long-held misconception that newborn piglets have a reduced sensation of pain - causes considerable pain and stress for the animals. Such practices have therefore been outlawed or are about to be forbidden in many jurisdictions.

While a variety of methods for anesthetizing piglets are known, for example general anesthesia using an inhalational anesthetic such as isoflurane or local anesthesia by injection of a local anesthetic, implementation of these methods on a large scale poses considerable challenges. Certain types of anesthetization may for example require expensive equipment such as inhalational anesthesia machines as well as the presence of a veterinarian in some countries. Furthermore, these procedures may be time-consuming, may carry a substantial risk of causing detrimental side effects and may be prone to improper execution. The latter for example applies for local anesthetization of the piglet's scrotum, which may require a precise injection of an efficient local anesthetic at appropriate positions and in an appropriate amount in order to achieve sufficient pain relief. This may be difficult to achieve when the anesthetization is to be performed in an industrial setting on a large number of animals, in particular when the procedure is not to be executed by a trained veterinarian. Moreover, on a fundamental level it is not yet clear whether local anesthesia is even capable of completely eliminating pain during castration as required in some jurisdictions, see e.g. N. Abendschön et al., Animals 2020, 10, 1752 and A. M. Saller et al., PLoS ONE 15(7): e0236742 (2020*).*

### SUMMARY OF THE INVENTION

It is thus an object of the invention to provide a device for administering a local anesthetic for castration of a piglet that is easy to handle and facilitates an efficient and proper local anesthetization of the piglet.

This object is met by a cannula for administering a local anesthetic for castration of a piglet according to claim 1. Embodiments of the present invention are detailed in the dependent claims.

The cannula for administering a local anesthetic for castration of a piglet according to the invention comprises a hollow shaft that extends from a proximal end to a distal end. The distal end of the hollow shaft comprises a needle tip for inserting the distal end through a scrotal skin of a scrotum of the piglet into a testicle of the piglet. A depth reference is arranged along the hollow shaft, wherein the depth reference indicates a depth up to which the hollow shaft is to be inserted into the scrotum. The hollow shaft comprises a plurality of openings for releasing the local anesthetic from the hollow shaft through each of the openings simultaneously. The plurality of openings comprises a distal opening, a distal lateral opening, and a proximal lateral opening. The distal opening is arranged at the distal end of the hollow shaft and each of the distal and proximal lateral openings is arranged on one or more side faces of the hollow shaft. A center of the distal lateral opening is arranged at a first distance z₁ from the needle tip, the first distance being between 2.5 mm and 6.0 mm. The proximal lateral opening is arranged between the depth reference and the distal lateral opening. A center of the proximal lateral opening is arranged at a second distance z₂ from the depth reference, the second distance being between 0.5 mm and 2.0 mm.

The hollow shaft may for example comprise an outer wall surrounding a channel that is in fluid communication with the plurality of openings. The outer wall may for example comprise one or more side walls, each of which may e.g. extend along a longitudinal direction of the hollow shaft, the longitudinal direction extending from the proximal end of the hollow shaft to its distal end. The hollow shaft may further comprise an inlet, e.g. at the proximal end, that is in fluid communication with the openings, e.g. via the channel, and configured to receive the local anesthetic. The inlet may be configured to be connected to a fluid reservoir containing the local anesthetic, for example a syringe, either directly or indirectly via an intermediate coupling member such as a connector. In some embodiments, the cannula may also comprise the connector and/or the fluid reservoir, e.g. as detailed below.

The openings may for example extend through the outer wall of the hollow shaft, thereby providing a fluid communication between the channel and the surroundings of the hollow shaft, e.g. the surrounding tissue. This allows for simultaneously releasing the local anesthetic from the hollow shaft through each of the openings, e.g. by providing the local anesthetic in fluid form at the proximal end of the hollow shaft and generating a flow of the local anesthetic along the hollow shaft. A first part of the flow may leave the hollow shaft through the proximal lateral opening, a second part of the flow may leave the hollow shaft through the distal lateral opening and a third part of the flow may leave the hollow shaft through the distal opening. While maintaining the flow, the local anesthetic may thus be released through each of the openings to the surrounding tissue at the same time. When generating the flow, the flow may first reach the proximal lateral opening, then the distal lateral opening and finally the distal opening, i.e. there may be a small time delay between the onset of the partial flows through the respective openings. Preferably, the hollow shaft is already filled with fluid prior to insertion into the scrotum, e.g. by releasing a small amount of local anesthetic from the openings before inserting the needle tip through the scrotal skin, in order to avoid injection of air into the scrotum. In this case, the partial flows through the respective openings may start simultaneously without a time delay when applying a pressure at the proximal end.

The one or more side faces of the hollow shaft, on which the distal and proximal lateral openings are arranged, may for example be surfaces of the outer wall, e.g. surfaces of the side walls forming the outer wall, that extend along the longitudinal direction of the hollow shaft. The center of an opening may for example be the centroid or geometric center of the respective opening or may be a center point half way between a proximal edge and a distal edge of the respective opening. In some embodiments, one or more of the openings may comprise a plurality of sub-openings, e.g. as detailed below, wherein the center of the respective opening may for example be the combined geometric center or center point of the plurality of sub-openings.

The first and second distances may for example be measured along the longitudinal direction, e.g. along a straight line connecting the proximal and distal ends of the hollow shaft. In other words, the first and second distances may correspond to a length of a projection of a vector connecting the respective points, e.g. the center of the distal lateral opening to the needle tip and the center of the proximal lateral opening to the depth reference, respectively, onto the longitudinal direction. The first distance z₁ is between 2.5 mm and 6.0 mm, in some examples between 2.5 mm and 5.0 mm, in one example between 3.0 mm and 3.5 mm. The second distance z₂ is between 0.5 mm and 2.0 mm, in some examples between 0.75 mm and 1.75 mm, in one example between 1.0 mm and 1.5 mm. The second distance z₂ may for example be measured between the center of the proximal lateral opening and the portion or point of the depth reference that is closest to the proximal lateral opening.

The depth reference provides a reference point that facilitates insertion of the hollow shaft to a desired depth, e.g. such that the distal opening and the distal lateral opening are arranged or received in the testicle of the piglet and the proximal lateral opening is arranged or received in the subcutaneous tissue of the scrotum. The hollow shaft may for example be inserted into the scrotum to a depth at which the depth reference is at the level of or in contact with an outer surface of the scrotal skin. The depth reference may for example be or comprise a visible structure or feature along the hollow shaft, for example a depth marking such as a visible line, a protrusion or a groove on one or more side faces of the hollow shaft. Preferably, the depth reference is configured to prevent insertion of the hollow shaft beyond the desired depth. The depth reference may in particular be or comprise a contact surface that is configured to come in contact with an outer surface of the scrotal skin when the hollow shaft is inserted into the scrotum as detailed below. The depth reference may for example be arranged at the proximal end of the hollow shaft or between the proximal end and the proximal lateral opening, i.e. such that the proximal lateral opening is arranged between the depth reference and the distal end of the hollow shaft.

The arrangement of the plurality of openings is specifically adapted to the anatomy of newborn piglets, in particular piglets that are between 1 day and 7 days old, as well as to the castration procedure in order to facilitate an efficient and proper local anesthetization in a single shot without having to re-position the cannula. In particular, the positions of the openings are chosen such that when the distal end of the hollow shaft is inserted into a testicle of a piglet both the distal opening and the distal lateral opening may be arranged within the testicle, e.g. with the distal opening pointing towards the spermatic cord, while the proximal lateral opening is arranged in the subcutaneous tissue of the scrotum. Thereby, an intratesticular and subcutaneous or subscrotal injection of local anesthetic maybe performed simultaneously by releasing the local anesthetic from the plurality of openings. This may allow for providing pain relief both during a skin incision for opening the scrotal tissue and during the severing of the spermatic cord. The inventors have observed that by carefully adapting the arrangement of the openings satisfactory anesthesia may be achieved for castration even using a local anesthetic only or using a local anesthetic in combination with an analgesic.

Conventional methods for local anesthetization using a conventional cannula with a single opening (at the distal end of the hollow shaft) may e.g. involve two separate injections, one into the testicle for depositing the local anesthetic intratesticularly and another one into the subcutaneous tissue of the scrotum for depositing the local anesthetic subscrotally, see e.g. N. Abendschön et al., Animals 2020, 10, 1752*.* Alternatively, a single two-step injection may be used, which may involve first positioning the single opening (at the distal end of the hollow shaft) of the conventional cannula in the testicle for depositing the local anesthetic intratesticularly and subsequently depositing the local anesthetic subscrotally while withdrawing the cannula. This may lead to varying results with regard to both the regions in which the local anesthetic is injected and the amount of local anesthetic that is injected, making the reliable injection of the local anesthetic challenging and highly dependent on the skill and practice of the respective person performing the procedure. While it may be possible to achieve satisfactory anesthesia under controlled conditions in a laboratory or veterinarian clinic, this may not be the case under real-world conditions in an agricultural setting. In contrast, the cannula according to the present invention allows for a simplified placement of the cannula while at the same time ensuring the deposition of an appropriate amount of local anesthetic, e.g. by adapting the cross-sectional area of the openings accordingly. This may facilitate the handling of the cannula and may enable administration of the local anesthetic by non-veterinarians, making the cannula according to the invention suitable for large-scale use in stock farming.

In a preferred embodiment, the depth reference comprises a contact surface that is arranged along the hollow shaft and configured to come in contact with an outer surface of the scrotal skin of the piglet when the distal end of the hollow shaft is inserted into the testicle up to a desired insertion depth. The contact surface may for example extend in a direction that is tilted relative to the longitudinal direction, e.g. in a direction that is perpendicular or substantially perpendicular to the longitudinal direction. The contact surface may be a flat surface or may be a curved surface. The contact surface may in particular have a conical or substantially conical shape, wherein an opening angle or aperture of the cone may for example be between 90° and 180°. In other examples, the contact surface may e.g. be a spherical segment or a substantially spherical segment, in particular a spherical cap or a substantially spherical cap, for example a hemisphere. A width or a diameter of the contact surface perpendicular to the longitudinal direction may be larger than a width or a diameter of the hollow shaft, for example at least two times, preferably at least three times, in one example at least five times as large as the width or diameter of the hollow shaft, e.g. to prevent the contact surface from entering into the scrotal skin.

The contact surface may for example be arranged at the proximal end of the hollow shaft or between the proximal end and the proximal lateral opening, i.e. such that the proximal lateral opening is arranged between the contact surface and the distal end of the hollow shaft. The center of the proximal lateral opening is arranged at the second distance z₂ from the contact surface, wherein the second distance z₂ is between 0.5 mm and 2.0 mm, in some examples between 1.25 mm and 1.75 mm, in one example between 1.0 mm and 1.5 mm. The second distance z₂ may for example be measured between the center of the proximal lateral opening and the portion or point of the contact surface that is closest to the proximal lateral opening A distance between the contact surface and the needle tip, corresponding to a maximum depth up to which the hollow shaft can be inserted into the scrotum, may for example be between 5.5 mm and 13.5 mm, preferably between 6.5 mm and 12.5 mm, most preferably between 7.5 mm and 11.5 mm. Providing the contact surface may facilitate inserting the distal end of the hollow shaft to a well-defined depth and may ensure the correct placement of the openings within the scrotum, in particular the placement of the proximal lateral opening in the subcutaneous tissue.

In some embodiments, the depth reference may comprise one or more depth markings along the hollow shaft instead of or in addition to the contact surface, e.g. a visible line, a protrusion, and/or a groove at a position up to which the hollow shaft is to be inserted into the scrotum. The one or more depth markings may be arranged at the second distance z₂ from the proximal lateral opening, e.g. at the proximal end of the hollow shaft or between the proximal lateral opening and the proximal end of the hollow shaft.

In some embodiments, the cannula further comprises a connector that is configured to be connected to a fluid reservoir containing the local anesthetic for providing a fluid communication between the fluid reservoir and the hollow shaft. The connector may in particular be configured to receive a syringe for providing a fluid communication between the syringe and the hollow shaft. The connector may for example be arranged at the proximal end of the hollow shaft. Preferably, the contact surface is a distal end face of the connector, e.g. the surface of the connector that is closest to the distal end of the hollow shaft. In some examples, the hollow shaft and the connector or a part thereof may be formed as a single piece. In other examples, the hollow shaft and the connector may be formed as separate pieces and may be connected or attached to each other, e.g. by gluing, welding, and/or mechanical fastening.

In a preferred embodiment, the center of the proximal lateral opening is arranged at a third distance z₃ from the center of the distal lateral opening. The third distance may for example be between 2.5 mm and 5.0 mm, in some examples between 3.0 mm and 4.5 mm, in one example between 3.5 mm and 4.0 mm. In some examples, a ratio of the third distance z₃ to the first distance z₁, i.e. z₃/z₁, may be between 0.5 and 1.5, preferably between 0.75 and 1.25. A ratio of the second distance z₂ to the first distance z₁, i.e. z₂/z₁, may for example be between 0.20 and 0.50, in one example between 0.35 and 0.45.

Preferably, a cross-sectional area of the distal lateral opening is larger than a cross-sectional area of the proximal lateral opening. The cross-sectional area of the distal lateral opening may for example be between 1.5 times and 5.0 times, preferably between 1.5 times and 2.5 times as large as the cross-sectional area of the proximal lateral opening. The cross-sectional area of an opening may for example be defined as the smallest cross-sectional area of the respective opening perpendicular to a direction of flow of the local anesthetic through the respective opening. For an opening that comprises a plurality of sub-openings, the cross-sectional area of the respective opening may for example be defined as the sum of the cross-sectional areas of the sub-openings. The cross-sectional area of an opening may determine a flow rate of the local anesthetic through the respective opening and may thus be used for adapting the amount of local anesthetic that is injected through the respective opening. The cross-sectional area may for example be adapted to account for a decreasing fluid pressure or flow rate along the length of the hollow shaft and/or for a permeability of the surrounding tissue, both of which in general will be different at the respective locations of the openings in the inserted state of the cannula. The larger cross-sectional area of the distal lateral opening may for example allow for injecting an equal or larger amount of local anesthetic into the testicle as into the subcutaneous tissue in order to achieve sufficient pain relief for severing the spermatic cord, which constitutes the most painful step of the castration procedure.

A cross-sectional area of the distal opening may be larger than the cross-sectional area of the proximal lateral opening. The cross-sectional area of the distal opening may for example be between 2.0 times and 6.0 times, preferably between 2.0 times and 3.0 times as large as the cross-sectional area of the proximal lateral opening. Additionally or alternatively, the cross-sectional area of the distal opening may also be larger than the cross-sectional area of the distal lateral opening, e.g. between 1.1 times and 2.0 times, in some examples between 1.1 times and 1.5 times as large as the cross-sectional area of the distal lateral opening. In a preferred embodiment, a width, a diameter, and/or a cross-sectional area of the distal opening is equal to an inner width, an inner diameter and/or an inner cross-sectional area, respectively, of the hollow shaft, e.g. a width, a diameter, and/or a cross-sectional area of the channel within the hollow shaft.

The cross-sectional area of the proximal lateral opening may for example be between 0.01 mm² and 0.04 mm², preferably between 0.03 mm² and 0.04 mm². The cross-sectional area of the distal lateral opening may for example be between 0.04 mm² and 0.08 mm², preferably between 0.05 mm² and 0.07 mm². The cross-sectional area of the distal opening may for example be between 0.06 mm² and 0.20 mm², in some examples between 0.06 mm² and 0.12 mm², preferably between 0.07 mm² and 0.09 mm². The inner cross-sectional area of the hollow shaft may for example be between 0.06 mm² and 0.20 mm², in some examples between 0.06 mm² and 0.12 mm², preferably between 0.07 mm² and 0.09 mm². The hollow shaft may for example be a 24 Birmingham gauge shaft (24G, corresponding to an outer diameter of about 0.57 mm), a 25G shaft (corresponding to an outer diameter of about 0.51 mm) or a 26G shaft (corresponding to an outer diameter of about 0.46 mm).

In some embodiments, some or all of the openings may comprise a plurality of sub-openings, for example two or more sub-openings, which together form the respective opening. Each of the sub-openings may for example extend through the outer wall of the hollow shaft, thereby providing a fluid communication between the channel and the surroundings of the hollow shaft. The sub-openings of an opening may be arranged adjacent to each other, but may for example be arranged on different side faces of the hollow shaft and/or may be displaced or staggered with respect to each other along the longitudinal direction. The sub-openings may for example be arranged with an equidistant spacing along the longitudinal direction and/or in the azimuthal direction, e.g. spaced equidistantly along a circumference of the hollow shaft. This may e.g. facilitate a homogeneous deposition of the local anesthetic and/or increase the mechanical stability of the cannula.

In some embodiments, the proximal lateral opening comprises a first proximal lateral sub-opening and a second proximal lateral sub-opening. The first and second proximal lateral sub-openings may be arranged on opposite side faces of the hollow shaft, e.g. rotated by 180° around the longitudinal direction. The second proximal lateral sub-opening may be displaced between 0 mm and 1.0 mm, preferably between 0.25 mm and 0.75 mm, most preferably between 0.4 mm and 0.6 mm from the first proximal lateral sub-opening towards the distal end of the hollow shaft.

Additionally or alternatively, the distal lateral opening may comprise a first distal lateral sub-opening and a second distal lateral sub-opening. The first and second distal lateral sub-openings may be arranged on opposite side faces of the hollow shaft, e.g. rotated by 180° around the longitudinal direction. The second distal lateral sub-opening may be displaced between 0 mm and 1.0 mm, preferably between 0.25 mm and 0.75 mm, most preferably between 0.4 mm and 0.6 mm from the first distal lateral sub-opening towards the distal end of the hollow shaft.

In some embodiments, the first proximal and distal lateral sub-openings are arranged on a first side face of the hollow shaft and the second proximal and distal lateral sub-openings are arranged on a second side face of the hollow shaft opposite to the first side face. In other words, the sub-openings of the proximal and distal lateral openings may be arranged in a zig-zag pattern along the longitudinal direction. In other embodiments, the sub-openings of the proximal and distal lateral openings may be distributed along the circumference of the hollow shaft. For example, each sub-opening may be displaced by 90° along the azimuthal direction relative to the adjacent sub-opening along the longitudinal direction.

In some embodiments, the proximal lateral opening and the distal lateral opening are arranged on opposite side faces of the hollow shaft. For example, each of the proximal and distal lateral openings may comprise a single opening, wherein the two openings may e.g. be rotated by 180° around the longitudinal direction with respect to each other. In another example, one or both of the proximal and distal lateral openings may comprise a plurality of sub-openings, wherein the proximal lateral opening or the sub-openings of the proximal lateral opening are arranged on a first side face of the hollow shaft and the distal lateral opening or the sub-openings of the distal lateral opening are arranged on a second side face of the hollow shaft opposite to the first side face.

Each of the proximal and distal lateral openings or sub-openings may for example extend through a side wall of the hollow shaft at an angle between 85° and 95° to the longitudinal direction extending from the proximal end of the hollow shaft to its distal end. In other examples, some or all of the proximal and distal lateral openings or sub-openings may extend at a smaller angle to the longitudinal direction, e.g. such that a direction of flow through the respective opening comprises a component in the longitudinal direction towards the distal end of the hollow shaft. Some or all of the proximal and distal lateral openings or sub-openings may for example extend at an angle between 15° and 85°, in some examples between 30° and 60° to the longitudinal direction.

In a preferred embodiment, the distal opening extends through a distal end face of the hollow shaft along the longitudinal direction extending from the proximal end of the hollow shaft to its distal end. The distal end face of the hollow shaft may for example be a surface or a plane extending between the side faces or side walls of the hollow shaft at its distal end. In some examples, the distal opening may cover or extend over the entire end face of the hollow shaft apart from portions of the end face formed by side walls of the hollow shaft. For example, the distal end of the hollow shaft may be open such that the channel within the hollow shaft extends through the distal end face into the surroundings of the hollow shaft, i.e. a cross-section of the distal opening may be equal to a cross-section of the channel. In some embodiments, the distal end face of the hollow shaft is chamfered to form the needle tip, e.g. by cutting off the hollow shaft at an acute angle to the longitudinal direction. Preferably, the distal end face extends at an angle between 10° and 35° to the longitudinal direction, in some examples at an angle between 10° and 20°. A distance from a center of the distal opening to the needle tip may for example be between 0.0 mm and 1.5 mm, e.g. as a result of the chamfer or bevel of the distal end face. In some examples, the distal end face of the hollow shaft may comprise a two-face or three-face bevel to form the needle tip.

In some embodiments, the hollow shaft is a cylindrical tube, wherein an outer diameter of the tube may for example be between 0.4 mm and 0.6 mm and an inner diameter of the tube may for example be between 0.25 mm and 0.4 mm. The hollow shaft may for example be a 24 Birmingham gauge shaft (24G, corresponding to an outer diameter of about 0.57 mm), a 25G shaft (corresponding to an outer diameter of about 0.51 mm) or a 26G shaft (corresponding to an outer diameter of about 0.46 mm). In some examples, the hollow shaft may have a constant width or a constant diameter from the proximal end to the distal end. In other examples, the width or diameter of the hollow shaft may decrease form the proximal end to the distal end, wherein the aforementioned parameter ranges may e.g. refer to the extent of the hollow shaft at its center and/or at the proximal end. The hollow shaft may for example have a length between 5.5 mm and 16.5 mm, preferably between 5.5 mm and 13.5 mm, most preferably between 7.5 mm and 11.5 mm. The length of the hollow shaft may for example be measured between the proximal end of the hollow shaft and the needle tip. A length in this range may for example facilitate placing the distal end of the hollow shaft in the testicle of the piglet in the vicinity of the spermatic cord, e.g. by inserting the hollow shaft into the scrotum up to the depth reference.

The invention further provides a method of administering a local anesthetic to a scrotum of a piglet using the cannula according to one of the embodiments described herein. The method comprises inserting the distal end of the hollow shaft through a scrotal skin of the piglet into a testicle of the piglet and releasing the local anesthetic through the plurality of openings while the distal end of the hollow shaft is arranged in the testicle.

Preferably, the hollow shaft is arranged such that the distal opening and the distal lateral opening are arranged within the testicle and the proximal lateral opening is arranged in a subcutaneous tissue of the scrotum. The hollow shaft may in particular be arranged such that the distal opening points towards a spermatic cord of the piglet. The center of the proximal lateral opening may for example be placed at a depth corresponding to the second distance z₂, e.g. between 0.5 mm and 2.0 mm, in some examples between 0.75 mm and 1.75 mm, in one example between 1.0 mm and 1.5 mm, from an outer surface of the scrotal skin. The center of the distal lateral opening may for example be placed at a depth between 3.0 mm and 7.0 mm, in some examples between 3.75 mm and 6.25 mm, in one example between 4.5 mm and 5.5 mm from the outer surface of the scrotal skin and the center of the distal opening at a depth between 4.5 mm and 13.5 mm, in some examples between 5.5 mm and 12.0 mm, in one example between 6.5 mm and 11.0 mm. In some embodiments, inserting the distal end of the hollow shaft comprises inserting the hollow shaft into the scrotum up to the depth reference, e.g. by bringing a contact surface of the cannula in contact with the outer surface of the scrotal skin or inserting the hollow shaft into the scrotum up to a depth marking. Preferably, the contact surface remains in contact with the outer surface of the scrotal skin while releasing the local anesthetic to ensure that the hollow shaft reaches and remains at a certain depth.

The local anesthetic may be released simultaneously from each of the plurality of openings, e.g. by generating a flow of the local anesthetic through the hollow shaft. In some examples, the amount or volume of local anesthetic released from the proximal lateral opening is between 20% and 200%, in some examples between 50% and 125% of the combined amount or volume of local anesthetic released from the distal lateral opening and the distal opening. The amount of local anesthetic released from the proximal lateral opening may be similar to the combined amount of local anesthetic released from the distal lateral opening and the distal opening and may for example be between 75% and 125%, in one example between 90% and 110% of the combined amount of local anesthetic released from the distal lateral opening and the distal opening. In other examples, the amount of local anesthetic released from the proximal lateral opening may be smaller than the combined amount of local anesthetic released from the distal lateral opening and the distal opening and may for example be between 20% and 60%, in one example between 35% and 55% of the combined amount of local anesthetic released from the distal lateral opening and the distal opening. The local anesthetic may for example comprise one or more substances selected from the group consisting of procaine, lidocaine, bupivacaine, mepivacaine, ropivacaine and dexmedetomidine, which may for example be dissolved in an aqueous solution. In some examples, the method may further comprise administering an analgesic, for example metamizole and/or a nonsteroidal anti-inflammatory drug such as meloxicam. The analgesic may for example be administered by a separate injection, in particular by an intramuscular injection, e.g. in the neck region of the piglet.

### LIST OF FIGURES

In the following, a detailed description of the invention and exemplary embodiments thereof is given with reference to the figures. The figures show schematic illustrations of
Fig. 1: a cannula for administering a local anesthetic for castration of a piglet according to an exemplary embodiment of the invention in a cross-sectional side view;
Fig. 2a: a cannula comprising a connector in accordance with an exemplary embodiment of the invention in side view;
Fig. 2b: the cannula of Fig. 2a in a perspective view
Fig. 3: a cannula inserted into a scrotum of a piglet according to an exemplary embodiment of the invention in side view;
Fig. 4: a flow chart of a method for administering a local anesthetic in accordance with an example;
Fig. 5: a cannula for administering a local anesthetic for castration of a piglet with a depth marking according to an exemplary embodiment of the invention in a cross-sectional side view;
Fig. 6a: a computed tomography (CT) image of a piglet anesthetized by single-step injection using a cannula in accordance with an exemplary embodiment of the invention; and
Fig. 6b: a CT image of a piglet anesthetized by two-step injection using a conventional cannula with a single opening (at the distal end of the hollow shaft).

### DESCRIPTION OF THE PREFERRED EMBODIMENTS

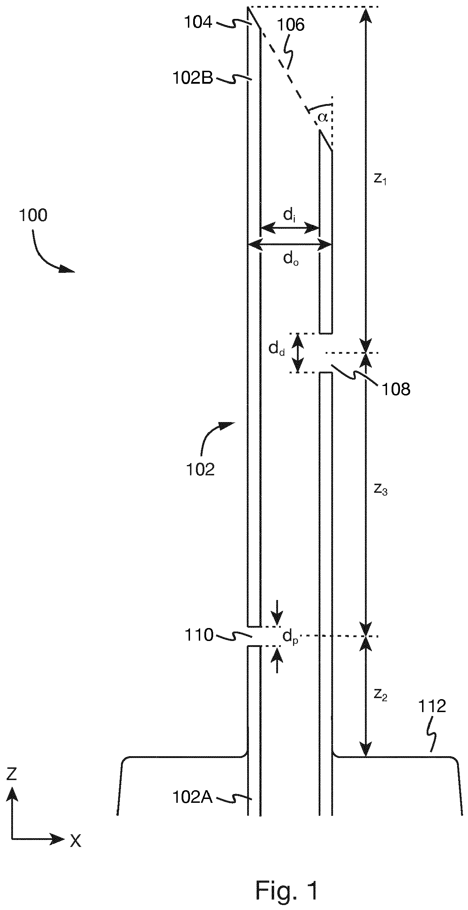

Fig. 1 shows a schematic illustration of a cannula 100 for administering a local anesthetic for castration of a piglet (not shown) according to an exemplary embodiment of the invention in a cross-sectional side view (not to scale). The cannula 100 comprises a hollow shaft 102 that extends from a proximal end 102A to a distal end 102B along a longitudinal direction, which is aligned with the Z axis of Fig. 1. The hollow shaft 102 may for example comprise or consist of metal, in particular stainless steel, and may e.g. have a length between 5.5 mm and 16.5 mm, preferably between 5.5 mm and 13.5 mm, most preferably between 7.5 mm and 11.5 mm, in one example between 7.5 mm and 10.5 mm. In the example of Fig. 1, the hollow shaft 102 is a straight, cylindrical tube comprising an outer wall with an outer diameter dₒ, which surrounds an inner channel with an inner diameter dᵢ. The outer diameter dₒ may for example be between 0.2 mm and 0.8 mm, in some examples between 0.4 mm and 0.6 mm (e.g. 0.5 mm corresponding to 25 Birmingham gauge (25G) in one example). The inner diameter dᵢ may for example be between 0.1 mm and 0.6 mm, in some examples between 0.25 mm and 0.4 mm, in one example between 0.25 mm and 0.35 mm. The hollow shaft 102 may for example be a 24 Birmingham gauge shaft (24G, corresponding to an outer diameter dₒ of about 0.57 mm), a 25G shaft (corresponding to an outer diameter dₒ of about 0.51 mm) or a 26G shaft (corresponding to an outer diameter dₒ of about 0.46 mm).

A distal end face of the hollow shaft 102 at the distal end 102B is chamfered or beveled, thereby forming a needle tip 104 at the distal end 102B for inserting the distal end 102B into a scrotum of the piglet. The distal end face extends at an angle α to the longitudinal direction, wherein α may for example be between 10° and 35° to facilitate inserting the hollow shaft 102 through the scrotal skin. A width of the needle tip 104 may for example be less than 20%, preferably less than 10% of the outer diameter dₒ. The width of the needle tip 104 may for example be the largest extent to the needle tip 104 in a plane perpendicular to the longitudinal direction, wherein the needle tip 104 may e.g. be the portion of the distal end 102B of the hollow shaft 102 that is the furthest away from the proximal end 102A and/or the portion of the distal end 102B having the smallest extent perpendicular to the longitudinal direction. In a preferred embodiment, the distal end face of the hollow shaft 102 has a three-face bevel to form the needle tip 104. The channel extends from the proximal end 102A along the length of the hollow shaft 102 and through the distal end face of the hollow shaft 102, at which the channel terminates at a distal opening 106 indicated by the corresponding dashed line in Fig. 1. The distal opening 106 at the distal end 102B of the hollow shaft 102 provides a fluid communication to the tissue surrounding the distal end 102B, e.g. a testicle of the piglet. In the example of Fig. 1, the distal opening 106 has a circular cross-section perpendicular to the longitudinal direction with a diameter corresponding to the inner diameter dᵢ.

In addition to the distal opening 106, the hollow shaft 102 comprises two further openings, namely a distal lateral opening 108 and a proximal lateral opening 110, which are arranged between the proximal and distal ends 102A, 102B on two opposite side faces of the hollow shaft 102. The proximal and distal lateral openings 110, 108 extend through the respective side wall of the hollow shaft 102, e.g. at an angle between 85° and 95° to the longitudinal direction, and thus provide additional fluid paths from the channel within the hollow shaft 102 to the surrounding tissue. Thereby, an anesthetic fluid provided at the proximal end 102A, e.g. from a fluid reservoir (not shown) such as a syringe connected thereto, may be released from the hollow shaft 102 through each of the plurality of openings 106, 108, 110 to the surrounding tissue simultaneously. The proximal and distal lateral openings 110, 108 may for the example have been formed by laser drilling.

A center of the distal lateral opening 108 is arranged at a first distance z₁ from the needle tip 104, wherein the first distance z₁ is measured along the longitudinal direction and the center of an opening corresponds to the geometric center of the respective opening as illustrated by the corresponding dotted lines in Fig. 1. The first distance z₁ is between 2.5 mm and 6.0 mm, preferably between 3.0 mm and 3.5 mm, e.g. such that both the distal opening 106 and the distal lateral opening 108 may be received within the testicle of a piglet in an inserted state of the cannula.

A center of the proximal lateral opening 110 is arranged at a third distance z₃ from the center of the distal lateral opening 108, wherein the third distance z₃ is also measured along the longitudinal direction between the geometric centers of the respective openings 108, 110. The third distance z₃ may for example be between 2.5 mm and 5.0 mm, preferably between 3.5 mm and 4.0 mm, e.g. such that the proximal lateral opening 108 may be arranged within the subcutaneous tissue of the scrotum when the distal opening 106 and the distal lateral opening 108 are received within the testicle of the piglet in an inserted state of the cannula. A ratio of the third distance z₃ to the first distance z₁, i.e. z₃/z₁, may for example be between 0.5 and 1.5, preferably between 0.75 and 1.25.

Each of the proximal and distal lateral openings 110, 108 has an elliptical shape, in particular a circular shape. The proximal lateral opening 110 may for example have a diameter or a length along the longitudinal direction dₚ between 0.05 mm and 0.3 mm, in one example between 0.15 mm and 0.25 mm. The distal lateral opening 108 may for example have a diameter or a length along the longitudinal direction d_{d} between 0.1 mm and 0.4 mm, in one example between 0.25 mm and 0.30 mm. The lengths dₚ and d_{d} may in particular be chosen such that a cross-sectional area of the distal lateral opening 108 is between 1.5 times and 5.0 times, preferably between 1.5 times and 2.5 times as large as a cross-sectional area of the proximal lateral opening 110 and a cross-sectional area of the distal opening 106 is between 2.0 times and 6.0 times, preferably between 2.0 times and 3.0 times as large as the cross-sectional area of the proximal lateral opening 110. In a preferred example, the cross-sectional area of the proximal lateral opening 110 is between 0.03 mm² and 0.04 mm², the cross-sectional area of the distal lateral opening 108 is between 0.05 mm² and 0.07 mm² and the cross-sectional area of the distal opening 106 is between 0.07 mm² and 0.09 mm². By providing openings with an increasing size along the longitudinal direction, i.e. from the proximal end 102A to the distal end 102B, the smaller permeability of the intratesticular tissue as compared to the subscrotal tissue as well as a decreasing fluid pressure or flow rate along the length of the hollow shaft 102 can be accounted for, e.g. to inject predetermined amounts of local anesthetic into the testicle and the subscrotal tissue.

The amount of local anesthetic released from the proximal lateral opening 110 may for example be between 20% and 200%, in some examples between 50% and 125% of the combined amount or volume of local anesthetic released from the distal lateral opening 108 and the distal opening 106. In some examples, a similar amount of local anesthetic may be injected into the testicle and the subscrotal tissue. The amount of local anesthetic released from the proximal lateral opening 110 may for example be between 75% and 125%, in one example between 90% and 110% of the combined amount or volume of local anesthetic released from the distal lateral opening 108 and the distal opening 106. In other examples, a smaller amount of local anesthetic may be injected into the subscrotal tissue than into the testicle. The amount of local anesthetic released from the proximal lateral opening 110 may for example be between 20% and 60%, in one example between 35% and 55% of the combined amount or volume of local anesthetic released from the distal lateral opening 108 and the distal opening 106.

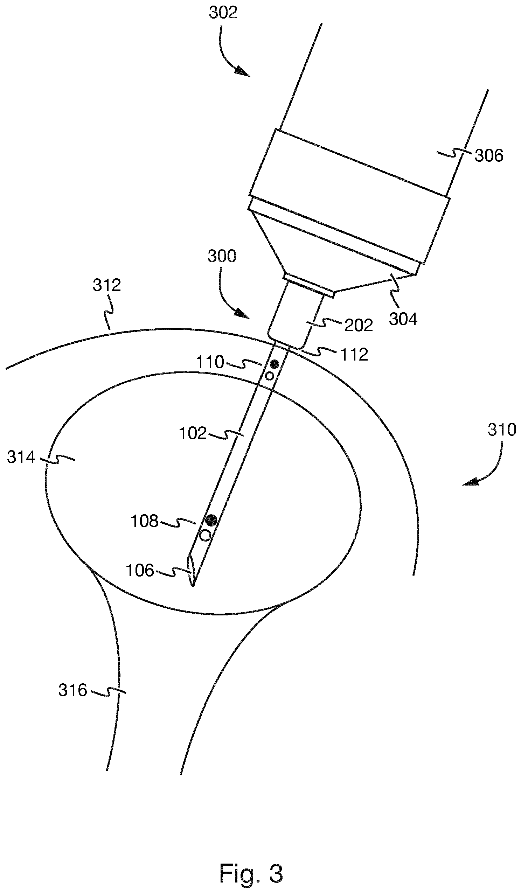

The cannula 100 further comprises a contact surface 112 that is arranged along the hollow shaft 102 between the proximal lateral opening 110 and the proximal end 102A. The contact surface 112 extends outwards from an outer circumference of the hollow shaft 102 and is substantially perpendicular to the longitudinal direction in the example of Fig. 1. The contact surface is configured to come in contact with an outer surface of the scrotal skin of the piglet when the distal end 102B of the hollow shaft 102 is inserted into the testicle, thereby preventing inserting the hollow shaft 102 further into the scrotum, e.g. as detailed below with reference to Fig. 3. Accordingly, the contact surface 112 serves as a depth reference that indicates a depth up to which the hollow shaft 102 is to be inserted into the scrotum 310. The contact surface 112 maybe formed together with the hollow shaft 102 as a single piece or may be attached to the hollow shaft 102 later on. The contact surface 112 may for example comprise or consist of metal, glass, and/or plastic, e.g. a thermoplastic such as polypropylene. The proximal lateral opening 110 is arranged between the contact surface 112 and the distal end 102B of the hollow shaft 102 at a second distance z₂ from the contact surface 112. The second distance z₂ is between 0.5 mm and 2.0 mm, preferably between 1.0 mm and 1.5 mm, to ensure that the proximal lateral opening 110 is arranged in the subscrotal tissue when the contact surface 112 is in contact with the scrotal skin. A distance between the contact surface 112 and the needle tip 104, i.e. z₁ + z₂ + z₃, may for example be between 5.5 mm and 13.5 mm, preferably between 7.5 mm and 11.5 mm.

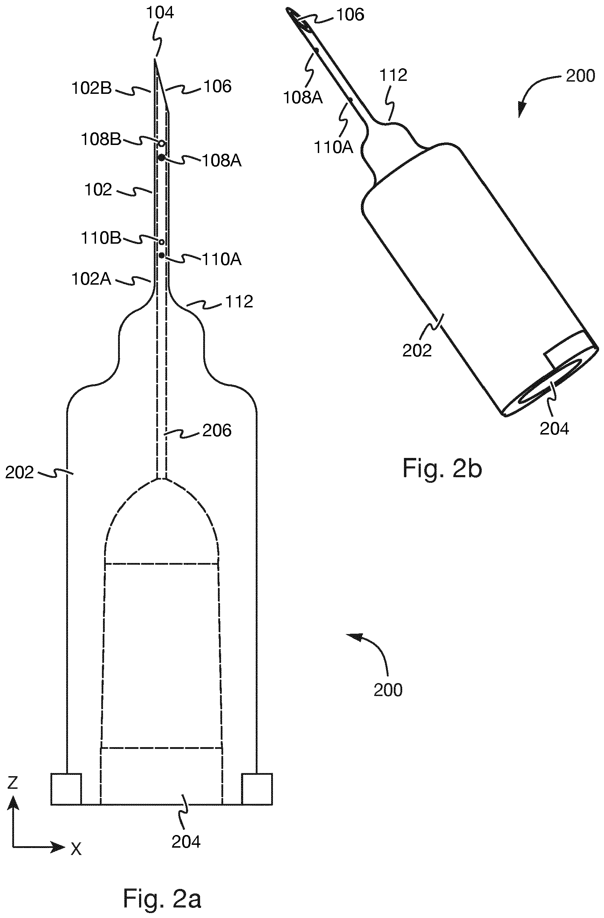

Figs. 2a and 2b depict a schematic illustration of a cannula 200 for administering a local anesthetic for castration of a piglet (not shown) in accordance with an exemplary embodiment of the invention. The cannula 200 is shown in Fig. 2a in side view (not to scale) and in Fig. 2b in a perspective view (not to scale). The cannula 200 is similar to the cannula 100 of Fig. 1, wherein corresponding elements are denoted using the same reference signs. In particular, the cannula 200 also comprises a hollow shaft 102 extending from a proximal end 102A to a distal end 102B. The hollow shaft 102 comprises a needle tip 104 at the distal end 102B for inserting the hollow shaft 102 into a scrotum of the piglet and a plurality of openings 106, 108, 110 for releasing the local anesthetic from the hollow shaft 102.

The plurality of openings comprise a distal opening 106 at the distal end 102B as well as a distal lateral opening 108 and a proximal lateral opening 110 arranged along the hollow shaft 102. Each of the proximal and distal lateral openings 110, 108 comprises a plurality of sub-openings. In the example of Fig. 2a, 2b, the proximal lateral opening 110 comprises a first proximal lateral sub-opening 110A and a second proximal lateral sub-opening 110B and the distal lateral opening 108 comprises a first distal lateral sub-opening 108A and a second distal lateral sub-opening 108B.

The first proximal lateral sub-opening 110A is arranged on a first side face of the hollow shaft 102, facing in the direction of view of Fig. 2a as indicated by the full circle. The second proximal lateral sub-opening 110B is arranged on a second side face of the hollow shaft 102 opposite to the first side face, i.e. facing away from the direction of view of Fig. 2a as indicated by the empty circle. The first and second proximal lateral sub-openings 110A, 110B are displaced with respect to each other along the longitudinal direction, which is aligned with the Z axis of Fig. 2a, such that the second proximal lateral sub-opening 110B is closer to the distal end 102B than the first proximal lateral sub-opening 110A. A distance between the centers of the first and second proximal lateral sub-openings 110A, 110B along the longitudinal direction may for example be between 0.1 mm and 1.0 mm, in some examples between 0.25 mm and 0.75 mm, e.g. 0.5 mm. The first and second proximal lateral sub-openings 110A, 110B have the same shape and size, e.g. a circular shape as in the example of Figs. 2a, 2b, wherein a diameter of each of the first and second proximal lateral sub-openings 110A, 110B may for example be between 0.05 mm and 0.2 mm, in some examples between 0.10 mm and 0.175 mm, e.g. 0.15 mm. Preferably, the diameter is chosen such that a cross-sectional area of the distal opening 106 is between 2.0 times and 6.0 times, in some examples between 2.0 times and 3.0 times as large as a combined cross-sectional area of the first and second proximal lateral sub-openings 110A, 110B. In the example of Fig. 2a, the diameter of the distal opening 106 corresponds to the inner diameter of the hollow shaft 102, which may for example be between 0.25 mm and 0.4 mm, in one example between 0.25 mm and 0.35 mm. The hollow shaft 104 may for example be a 24 Birmingham gauge to 26 Birmingham gauge shaft, e.g. a 25G shaft.

The first distal lateral sub-opening 108A is also arranged on the first side face of the hollow shaft 102, facing in the direction of view of Fig. 2a as indicated by the full circle. The second distal lateral sub-opening 108B is arranged on the second side face of the hollow shaft 102 opposite to the first side face, i.e. facing away from the direction of view of Fig. 2a as indicated by the empty circle. The first and second distal lateral sub-openings 108A, 108B are displaced with respect to each other along the longitudinal direction such that the second distal lateral sub-opening 108B is closer to the distal end 102B than the first distal lateral sub-opening 108A. A distance between the centers of the first and second distal lateral sub-openings 108A, 108B along the longitudinal direction may for example be between 0.1 mm and 1.0 mm, in some examples between 0.25 mm and 0.75 mm, e.g. 0.5 mm. The first and second distal lateral sub-openings 108A, 108B have the same shape and size, e.g. a circular shape as in the example of Figs. 2a, 2b. The first and second distal lateral sub-openings 108A, 108B are larger than the first and second proximal lateral sub-openings 110A, 110B, preferably such that the combined cross-sectional area of the first and second distal lateral sub-openings is between 1.5 times and 5.0 times, in some examples between 1.5 times and 2.5 times as large as the combined cross-sectional area of the first and second proximal lateral sub-openings 110A, 110B. A diameter of each of the first and second distal lateral sub-openings 110A, 110B may for example be between 0.1 mm and 0.3 mm, in some examples between 0.15 mm and 0.25 mm, e.g. 0.20 mm.

The cannula 200 further comprises a connector 202, which is arranged at the proximal end 102A of the hollow shaft 102. The connector 202 is configured to be connected to a fluid reservoir (not shown) containing the local anesthetic to provide a fluid communication between the fluid reservoir and the hollow shaft 102. In the example of Fig. 2a, 2b, the connector 202 comprises an insertion opening 204 that is configured to receive a connecting portion of a syringe (not shown) containing the local anesthetic, e.g. similar as shown in Fig. 3. The connector 202 further comprises a connecting channel 206 providing a fluid communication between the insertion opening 204 and the hollow shaft 102. The connector 202 or parts thereof, in particular the connecting channel 206, may be formed together with the hollow shaft 102 as a single piece. The connector 202 may for example comprise or consist of metal, glass, and/or plastic, e.g. a thermoplastic such as polypropylene. The connector 202 may comprise means for fixing the connecting portion of the syringe in the insertion opening 204 in order to provide a leak-free connection, e.g. a thread into which the connecting portion can be screwed or an appropriately shaped insertion opening 204 into which the connecting portion can be interference-fitted. Preferably, the connector 202 is formed in accordance with a common fluid fitting standard, e.g. the Luer taper (ISO 80369-7:2016).

A distal end face of the connector 202 adjacent to the proximal end 102A of the hollow shaft 102 forms a contact surface 112 extending along an outer circumference of the proximal end 102A and having a substantially conical shape, e.g. with an opening angle or aperture between 120° and 150°. An axis of the cone may be aligned with a longitudinal or central axis of the hollow shaft 102 with the tip of the cone pointing towards the distal end 102B. In other examples, the contact surface 112 may e.g. be a spherical segment or a substantially spherical segment, in particular a spherical cap or a substantially spherical cap, for example a hemisphere. The contact surface 112 is configured to come in contact with an outer surface of the scrotal skin (not shown) of the piglet and to prevent further insertion of the cannula 200, e.g. as detailed below with reference to Fig. 3. An outer diameter of the contact surface 112, e.g. at the base of the cone or spherical segment, may for example be between 2 times and 10 times, in one example between 3 times and 5 times as large as the outer diameter of the hollow shaft 102.

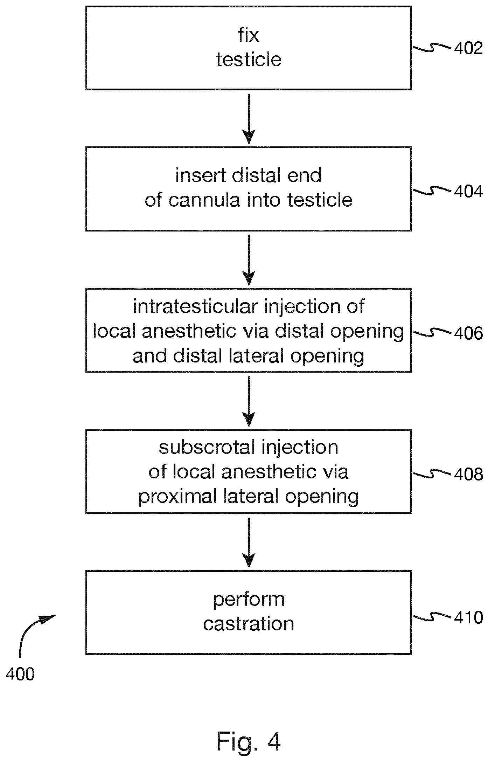

Fig. 3 depicts a schematic illustration of a cannula 300 according to an exemplary embodiment of the invention that has been inserted into a scrotum 310 of a piglet (not shown), e.g. as part of the method 400 described below with reference to Fig. 4. The cannula 300 is similar to the cannula 100 of Fig. 1 and the cannula 200 of Figs. 2a, 2b, wherein corresponding elements are denoted using the same reference signs. In particular, the cannula 300 also comprises a hollow shaft 102 having a plurality of openings 106, 108, 110 for releasing a local anesthetic from the hollow shaft 102. The hollow shaft 102 is connected to a connecting portion 304 of a syringe 302 via a connector 202, e.g. as described above for the cannula 200. The syringe 302 comprises a fluid reservoir 306, in which the local anesthetic may be provided in liquid form for example, wherein the fluid reservoir 306 is in fluid communication with the hollow shaft 102 via the connecting portion 304 and the connector 202. The syringe 302 may in particular be an automatic syringe that is configured to automatically release the entire content of the fluid reservoir 306 upon actuation. The syringe 302 may for example comprise a button or handle that is configured to trigger the release of an elastic member such as a spring upon actuation to advance a pumping member such as a piston along the fluid reservoir 306.

In the example of Fig. 3, the distal end of the hollow shaft 102 has been inserted through the scrotal skin 312 of the scrotum 310 into a testicle 314 of the piglet. The hollow shaft 102 has been inserted up to a depth at which a contact surface 112 formed by a distal end face of the connector 202 comes in contact with the scrotal skin 312 and prevents further insertion of the hollow shaft 102, thereby allowing for a precise control of the insertion depth. The length of the hollow shaft 102 from the contact surface 112 is chosen such that both the distal opening 106 and the distal lateral opening 108, which in this example comprises two sub-openings on opposite side faces of the hollow shaft 102 similar to the cannula 200, are arranged within the testicle 314, e.g. such that the distal opening 106 is adjacent to and faces the spermatic cord 316. The proximal lateral opening 110, which also comprises two sub-openings on opposite side faces of the hollow shaft 102, is at a distance from the contact surface 112 that is chosen such that the proximal lateral opening 110 is placed subcutaneously within the subscrotal tissue below the scrotal skin 312. In this way, local anesthetic provided by the syringe 302 may be released simultaneously into the testicle 314 and the subscrotal tissue with the relative amounts of local anesthetic released from the openings 106, 108, 110 being determined by the size of the respective opening 106, 108, 110 and the permeability of the surrounding tissue.

Fig. 4 depicts a flow chart of a method 400 of administering a local anesthetic to a scrotum of a piglet using a cannula according to one of the embodiments described herein. The method 400 may for example be executed with one of the cannulas 100, 200, 300 and will be described in the following using the cannula 300 of Fig. 3 as a non-limiting example for illustration purposes. The method 400 is not limited to the order of execution implied by the flow chart of Fig. 4. For example, parts of the method 400 may also be executed simultaneously at least in part, in particular steps 406 and 408. The method 400 may for example be executed for castrating a piglet, in particular a newborn piglet, e.g. of an age between 1 days and 7 days, but may also be executed for or as part of other surgical or non-surgical procedures.

The method 400 comprises, at step 402, fixing a testicle 314 of the piglet, e.g. by moving the testicle 314 caudally and fixing the testicle 314 between two fingers or in an appropriate fixation tool. Preferably, the scrotal skin 312 is stretched slightly, e.g. to prevent formation of wrinkles or folds above the testicle 314. This may for example be advantageous in settings in which the method 400 is not performed by a trained veterinarian as it greatly facilitates arranging the openings 106, 108, 110 of the hollow shaft 102 at the desired positions within the scrotum 310.

The method 400 further comprises, at step 404, inserting the distal end of the hollow shaft 102 through the scrotal skin 312 into the testicle 314, e.g. through a stretched portion of the scrotal skin 312 above the testicle 314. For this, the cannula 300 may be provided together with the syringe 302, wherein the cannula 300 may already have been attached to the syringe 302 prior to execution of the method 400 or may be attached to the syringe 302 as part of the method 400 prior to insertion. The reservoir 306 of the syringe 302 contains the local anesthetic, preferably in liquid form, wherein the local anesthetic may for example comprise at least one substance selected from the group consisting of procaine, lidocaine, bupivacaine, mepivacaine, ropivacaine and dexmedetomidine. In some embodiments, the local anesthetic may also comprise additional substances, for example aqua ad injectabilia (water for injection), sodium chloride, sodium hydroxide, sodium metabisulfite, hydrochloric acid, epinephrine and/or norepinephrine. Preferably, the hollow shaft 102 is completely filled with the local anesthetic prior to insertion into the scrotum 310, e.g. to avoid injection of air. For this, a flow of the local anesthetic through the hollow shaft 102 may be generated prior to insertion, e.g. until a small amount of the local anesthetic is released from the distal opening 106.

In step 404, the hollow shaft 102 may for example be arranged as illustrated in Fig. 3, i.e. the distal opening 106 and distal lateral opening 108 may be placed within the testicle 314, whereas the proximal lateral opening 110 is placed within the subcutaneous tissue of the scrotum 310. For this, the hollow shaft 102 may be advanced into the scrotum 310 until the contact surface 112 comes in contact with the scrotal skin 312. The center of the proximal lateral opening 110 may for example be placed at a depth between 1.0 mm and 1.5 mm below the outer surface of the scrotal skin. The center of the distal lateral opening 108 may for example be placed at a depth between 4.5 mm and 5.5 mm below the outer surface of the scrotal skin 312. The center of the distal opening 106 may for example be placed at a depth between 6.5 mm and 11.0 mm below the outer surface of the scrotal skin 312. Preferably, the distal end 102B of the hollow shaft 102 is arranged such that the distal end 102B is arranged adjacent to the spermatic cord 316 with the distal opening 106 facing the spermatic cord 316, e.g. as shown in Fig. 3.

Subsequently, in steps 406 and 408, the local anesthetic is released from the fluid reservoir 306 via the hollow shaft 102 through the plurality of openings 106, 108, 110 while the distal end 102B of the hollow shaft 102 is arranged in the testicle 314. Preferably, the contact surface 112 remains in contact with the scrotal skin 312 throughout the injection of the local anesthetic to ensure the correct placement of the openings 106, 108, The local anesthetic is released simultaneously from the proximal lateral opening 110 into the subscrotal tissue and from the distal lateral and distal openings 108, 106 into the testicle 314. Preferably, a smaller amount of local anesthetic is released into the subscrotal tissue than into the testicle 314. The fluid reservoir 306 may for example store between 0.4 ml and 2.0 ml of the local anesthetic, wherein e.g. between 0.1 ml and 0.8 ml of the local anesthetic is released via the proximal lateral opening 110 and e.g. between 0.2 ml and 1.5 ml of the local anesthetic is released via the distal lateral and distal openings 108, 106. In a preferred embodiment, the fluid reservoir 306 stores between 0.5 ml and 1.0 ml of the local anesthetic and the amount of local anesthetic released into the subscrotal tissue via the proximal lateral opening 110 is between 20% and 60%, in one example between 35% and 55% of the amount of local anesthetic released into the testicle 314 via the distal lateral and distal openings 108, 106. In one example, the fluid reservoir 306 stores between 0.6 ml and 0.7 ml of the local anesthetic, of which between 0.4 ml and 0.5 ml are released intratesticularly via the distal lateral and distal openings 108, 106 with 0.2 ml being released subscrotally via the proximal lateral opening After releasing the local anesthetic, e.g. after completely emptying the fluid reservoir 306, the hollow shaft 102 may be removed from the scrotum 310. Preferably, no local anesthetic is released from the openings 106, 108, 110 while withdrawing the cannula 300.

In some embodiments, the method 400 may also comprise performing a surgical or non-surgical procedure on the scrotum 310 in step 410. For example, the method 400 may comprise performing a castration in step 410. This may comprise opening the scrotal tissue and the tunica vaginalis by a skin incision, e.g. using a scalpel. Subsequently, the spermatic cord 316 may be severed and the testicle 314 may be removed from the scrotum 310. In some examples, the method 400 maybe executed repeatedly, e.g. to remove a second testicle 314.

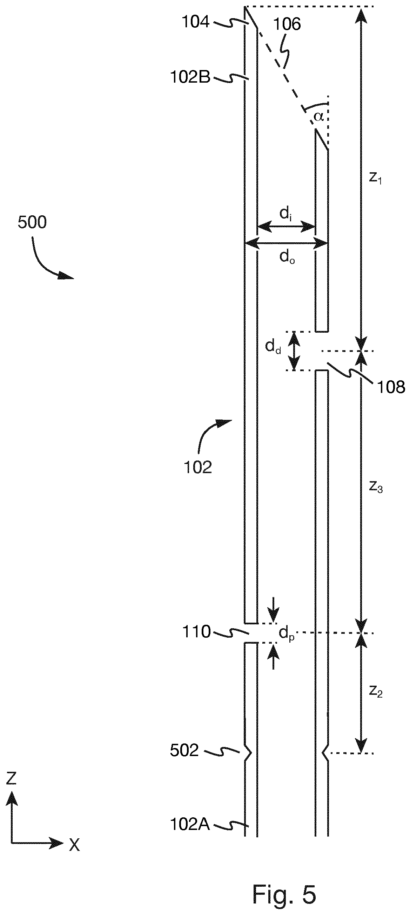

Fig. 5 depicts a schematic illustration of a cannula 500 for administering a local anesthetic for castration of a piglet according to another exemplary embodiment of the invention in a cross-sectional side view. The cannula 500 is similar to the cannula 100 of Fig. 1, wherein corresponding elements are denoted using the same reference signs. The cannula 500 also comprises a hollow shaft 102 with a plurality of openings 106, 108, Instead of the contact surface 112 of the cannula 100, the cannula 500 comprises a depth marking, in particular a groove 502 in the outer wall of the hollow shaft 102 as a depth reference. The groove 502 is arranged on side faces of the hollow shaft 102 at the second distance z₂ from the center of the proximal lateral opening 110 and may e.g. extend around the entire circumference of the hollow shaft 102. A width and a depth of the groove 502 may for example be chosen such that the groove 502 is easily visible to the naked eye, but does not compromise the mechanical stability and impermeability of the outer wall of the hollow shaft 102. In other examples, the cannula may comprise a visible line and/or a protrusion instead of or in addition to the groove 502.

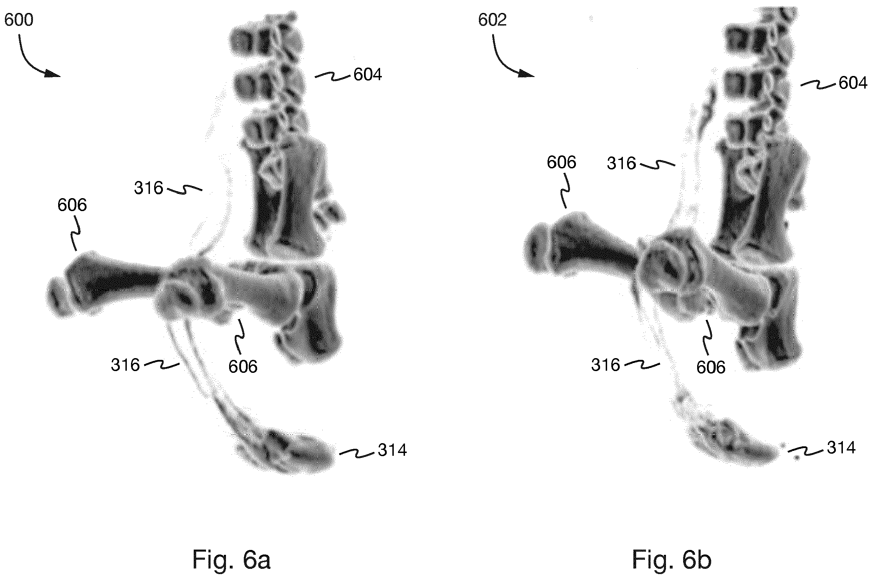

Figs. 6a and 6b depict computed tomography (CT) images of two piglets which have been anesthetized with a local anesthetic as a part of a comparative study performed by the inventors. Fig. 6a shows a CT image 600 of a piglet into the scrotum of which a local anesthetic has been injected by single step-injection using a cannula according to an exemplary embodiment of the invention similar to the cannula 200 of Fig. 2a, 2b. Fig. 6b shows a CT image 602 of a piglet into the scrotum of which a local anesthetic has been injected by two-step injection using a conventional cannula with a single opening at its distal end. For this, the single opening of the conventional cannula was first positioned in the testicle of the piglet for injecting the local anesthetic intratesticularly and subsequently, while withdrawing the cannula, the local anesthetic was also injected subscrotally. In both cases, an x-ray contrast agent was added to the local anesthetic in order to visualize the distribution of the local anesthetic in the piglet by computed tomography and the injections were administered by the same person, a trained veterinarian.

The CT images 600, 602 show the pelvic region of the piglets, wherein bony structures such as the vertebral column 604 and the femurs 606 of the piglets are clearly visible. In addition, the testicles 314 as well as the spermatic cords 316, which do not contain any bony structures, can be seen. This demonstrates that the local anesthetic can be deposited with the cannula according to the invention in a targeted fashion, allowing for an effective local anesthetization of the testicles 314 and the spermatic cords 316, e.g. to perform a castration. In particular, it should be noted that the resulting distribution of the local anesthetic in the image 600 of Fig. 6a is similar to the results achieved by the two-step injection with a conventional cannula when administered by a trained veterinarian (Fig. 6b). The latter, however, requires a very precise and careful handling of the cannula to properly inject the local anesthetic at the desired positions/depths and in the appropriate quantities. The cannula according to the invention, in contrast, ensures the correct positioning of the openings along the hollow shaft by means of the depth reference, e.g. the contact surface 112, wherein the quantities of the local anesthetic released from the openings are determined by the cross-sectional area of the respective opening. The local anesthetic can thus easily be administered even by untrained personnel, thereby allowing for large-scale deployment in an agricultural setting for example.

The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

100 - cannula
102 - hollow shaft
102A - proximal end of the hollow shaft 102
102B - distal end of the hollow shaft 102
104 - needle tip
106 - distal opening
108 - distal lateral opening
110 - proximal lateral opening
112 - contact surface
z₁ - first distance
z₂ - second distance
z₃ - third distance
dᵢ - inner diameter of the hollow shaft 102
dₒ - outer diameter of the hollow shaft 102
d_{d} -diameter of the distal lateral opening 108
dₚ -diameter of the proximal lateral opening 110
α - angle of distal end face of the hollow shaft 102
200 - cannula
108A - first distal lateral opening
108B - second distal lateral opening
110A - first proximal lateral opening
110B - second proximal lateral opening
202 - connector
204 - insertion opening
206 - connecting channel
300 - cannula
302 - syringe
304 - connecting portion
306 - fluid reservoir
310 - scrotum
312 - scrotal skin
314 - testicle
316 - spermatic cord
400 - method of administering a local anesthetic to a scrotum of a piglet
402 - step of fixing a testicle
404 - step of inserting the distal end of the hollow shaft into the testicle
406 - step of injecting the anesthetic intratesticularly via the distal lateral and distal openings
408 - step of injecting the anesthetic subscrotally via the proximal lateral opening
410 - step of performing castration
500 - cannula
502 - depth marking/groove
600 - computed tomography image of a piglet anesthetized by single-step injection using a cannula according to an embodiment of the invention
602 - computed tomography image of a piglet anesthetized by two-step injection using a conventional cannula with a single opening at its distal end
604 - vertebral column
606 - femur

## Claims

1. A cannula (100, 200, 300, 500) for administering a local anesthetic for castration of a piglet, the cannula (100, 200, 300) comprising a hollow shaft (102) extending from a proximal end (102A) to a distal end (102B), wherein:
the distal end (102B) of the hollow shaft (102) comprises a needle tip (104) for inserting the distal end (102B) through a scrotal skin (312) of a scrotum (310) of the piglet into a testicle (314) of the piglet;
a depth reference (112, 502) is arranged along the hollow shaft (102), the depth reference (112, 502) indicating a depth up to which the hollow shaft (102) is to be inserted into the scrotum (310); and
the hollow shaft (102) comprises a plurality of openings (106, 108, 110) for releasing the local anesthetic from the hollow shaft (102) through each of the openings (106, 108, 110) simultaneously,
the plurality of openings (106, 108, 110) comprising:
a distal opening (106) arranged at the distal end (102B) of the hollow shaft (102);
a distal lateral opening (108) arranged on a side face of the hollow shaft (102), wherein a center of the distal lateral opening (108) is arranged at a first distance (z₁) from the needle tip (104), the first distance (z₁) being between 2.5 mm and 6.0 mm; and
a proximal lateral opening (110) arranged on a side face of the hollow shaft (102), wherein the proximal lateral opening (110) is arranged between the depth reference (112, 502) and the distal lateral opening (108) and a center of the proximal lateral opening (110) is arranged at a second distance (z₂) from the depth reference (112, 502), the second distance (z₂) being between 0.5 mm and 2.0 mm.

2. The cannula (100, 200, 300) of claim 1, wherein the depth reference comprises a contact surface (112) that is configured to come in contact with an outer surface of the scrotal skin (312) of the piglet when the distal end (102B) of the hollow shaft (102) is inserted into the testicle (314).

3. The cannula (200, 300) of claim 2, further comprising a connector (202) arranged at the proximal end (102A) of the hollow shaft (102), the connector (202) being configured to receive a syringe (302) containing the local anesthetic for providing a fluid communication between the syringe (302) and the hollow shaft (102), wherein the contact surface (112) is a distal end face of the connector (202).

4. The cannula (100, 200, 300, 500) of any one of the preceding claims, wherein the center of the proximal lateral opening (110) is arranged at a third distance (z₃) from the center of the distal lateral opening (108), a ratio of the third distance (z₃) to the first distance (z₁) being between 0.5 and 1.5, preferably between 0.75 and 1.25, and/or the third distance (z₃) being between 3.0 mm and 4.5 mm.

5. The cannula (100, 200, 300, 500) of any one of the preceding claims, wherein a cross-sectional area of the distal lateral opening (108) is between 1.5 times and 5.0 times, preferably between 1.5 times and 2.5 times as large as a cross-sectional area of the proximal lateral opening (110).

6. The cannula (100, 200, 300, 500) of any one of the preceding claims, wherein a cross-sectional area of the distal opening (106) is between 2.0 times and 6.0 times, preferably between 2.0 times and 3.0 times as large as the cross-sectional area of the proximal lateral opening (110).

7. The cannula (100, 200, 300, 500) of any one of the preceding claims, wherein:
the cross-sectional area of the proximal lateral opening (110) is between 0.01 mm² and 0.04 mm², preferably between 0.03 mm² and 0.04 mm²;
the cross-sectional area of the distal lateral opening (108) is between 0.04 mm² and 0.08 mm², preferably between 0.05 mm² and 0.07 mm²; and
the cross-sectional area of the distal opening (106) is between 0.06 mm² and 0.12 mm², preferably between 0.07 mm² and 0.09 mm².

8. The cannula (200, 300) of any one of the preceding claims, wherein:
the proximal lateral opening (110) comprises a first proximal lateral sub-opening (110A) and a second proximal lateral sub-opening (110B), wherein the first and second proximal lateral sub-openings (110A, 110B) are arranged on opposite side faces of the hollow shaft (102) and the second proximal lateral sub-opening (110B) is displaced between 0.25 mm and 0.75 mm from the first proximal lateral sub-opening (110A) towards the distal end (102B) of the hollow shaft (102).

9. The cannula (200, 300) of any one of the preceding claims, wherein:
the distal lateral opening (108) comprises a first distal lateral sub-opening (108A) and a second distal lateral sub-opening (108B), wherein the first and second distal lateral sub-openings (108A, 108B) are arranged on opposite side faces of the hollow shaft (102) and the second distal lateral sub-opening (108B) is displaced between 0.25 mm and 0.75 mm from the first distal lateral sub-opening (108A) towards the distal end (102B) of the hollow shaft (102).

10. The cannula (200, 300) of claim 8 and 9, wherein the first proximal and distal lateral sub-openings (110A, 108B) are arranged on a first side face of the hollow shaft (102) and the second proximal and distal lateral sub-openings (110B, 108B) are arranged on a second side face of the hollow shaft (102) opposite to the first side face.

11. The cannula (100, 500) of any one of claims 1 to 7, wherein the proximal lateral opening (110) and the distal lateral opening (108) are arranged on opposite side faces of the hollow shaft (102).

12. The cannula (100, 200, 300, 500) of any one of the preceding claims, wherein each of the proximal and distal lateral openings (110, 108) or sub-openings (110A, 110B, 108A, 108B) extends through a side wall of the hollow shaft (102) at an angle between 85° and 95° to a longitudinal direction extending from the proximal end (102A) of the hollow shaft (102) to its distal end (102B).

13. The cannula (100, 200, 300, 500) of any one of the preceding claims, wherein the distal opening (106) extends through a distal end face of the hollow shaft (102) along the longitudinal direction extending from the proximal end (102A) of the hollow shaft (102) to its distal end (102B).

14. The cannula (100, 200, 300, 500) of claim 13, wherein the distal end face of the hollow shaft (102) is chamfered to form the needle tip (104) and extends at an angle (a) between 10° and 35° to the longitudinal direction.

15. The cannula (100, 200, 300, 500) of any one of the preceding claims, wherein the hollow shaft (102) is a cylindrical tube with an outer diameter (dₒ) between 0.4 mm and 0.6 mm and an inner diameter (dᵢ) between 0.25 mm and 0.4 mm and/or wherein the hollow shaft (102) has a length between 5.5 mm and 16.5 mm, preferably between 7.5 mm and 11.5 mm.
